# EUROPÄISCHE PATENTSCHRIFT

(11) **EP 0 922 686 B1**
(45) Veröffentlichungstag und Bekanntmachung des Hinweises auf die Patenterteilung: **06.02.2002**
(21) Anmeldenummer: 98122669.9
(22) Anmeldetag: 30.11.1998
(51) Int. Cl.: C07C 13/04

(54) **Verfahren zur Herstellung von Ethinylcyclopropan**
Process for the preparation of ethynylcyclopropane
Procédé pour la préparation de l'éthynylcyclopropane

(30) Priorität: 10.12.1997 CH 284297
(43) Veröffentlichungstag der Anmeldung: 16.06.1999
(73) Patentinhaber: LONZA A.G., CH-4002 Basel (CH)
(72) Erfinder: Michel, Dominique Dr., 3952 Susten (CH); Hanselmann, Paul Dr., 3902 Brig-Glis (CH)

(56) Entgegenhaltungen:
- WO-A-96/22955
- WO-A-98/40333
- E. TASKINEN: "Thermodynamics of Vinyl Ethers. VIII. A Study of the Relative Ring Strain Energies and Relative Ring Entropies of Three- to Eight-membered Carbocyclic Rings with One or No sp2-Hybridized Carbon Atoms" ACTA CHEMICA SCANDINAVICA. SERIES B - ORGANIC CHEMISTRY AND BIOCHEMISTRY, Bd. 28, Nr. 3, 1974, Seiten 357-366, XP002095240 COPENHAGEN (DK)

## Beschreibung

Die vorliegende Erfindung betrifft ein Verfahren zur Herstellung von Ethinylcyclopropan der Formel

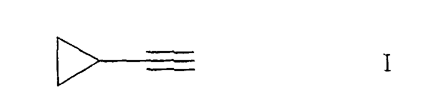

Ethinylcyclopropan ist ein Zwischenprodukt in der Synthese von pharmazeutischen Wirkstoffen, beispielsweise von antiviralen Mitteln zur Bekämpfung von HIV-Infektionen (WO 96/22955).

Eine bekannte Synthese von Ethinylcyclopropan geht von 1-Brom-3-chlorpropan aus, welches mit Natriumacetylid zu 5-Chlor-1-pentin umgesetzt wird. Dieses wird mit *n*-Butyllithium zu der gewünschten Verbindung cyclisiert. Ein Nachteil dieses Verfahrens liegt darin, dass es tiefe Temperaturen erfordert. Ein weiteres bekanntes Verfahren (WO 96/22955), geht von Cyclopropylmethylketon aus, welches zunächst in das (1,1-Dichlorethyl)cyclopropan übergeführt wird. Letzteres ergibt nach Eliminierung von 2 Molekülen Chlorwasserstoff das gewünschte Produkt. Nachteilig an diesem Verfahren ist, dass die Herstellung des geminalen Dichlorids drastische Bedingungen (z.B. PCl₅) erfordert. Ausserdem liefern beide genannten Verfahren mindestens zwei Mol Halogenidabfall pro Mol Produkt.

Aufgabe der vorliegenden Erfindung war daher, ein alternatives Verfahren zur Herstellung von Ethinylcyclopropan zur Verfügung zu stellen, welches keine tiefen Temperaturen erfordert und weniger Abfall liefert.

Erfindungsgemäss wird diese Aufgabe durch das Verfahren nach Patentanspruch 1 gelöst.

Es wurde gefunden, dass aus dem (1,1-Dimethoxyethyl)cyclopropan der Formel 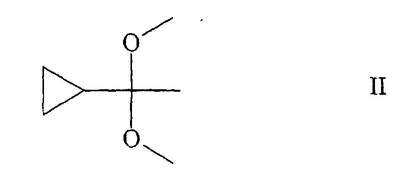 in zwei Stufen Methanol eliminiert werden kann, wobei zunächst der Enolether (1-Methoxyethenyl)cylopropan der Formel 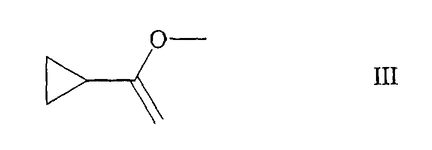 und dann das gewüschte Produkt (I) entsteht.
Das (1,1-Dimethoxyethyl)cyclopropan kann auf bekannte Weise aus dem kommerziell erhältlichen Cyclopropylmethylketon und Trimethylorthoformiat hergestellt werden (Acta Chem. Scand B28 (1974) No.3, 357-366).

Die erste Stufe des erfindungsgemässen Verfahrens wird vorzugsweise unter heterogener Katalyse durchgeführt. Dies hat den Vorteil, dass der Katalysator leicht entfernt und gegebenenfalls wiederverwendet werden kann.

Als heterogener Katalysator ist Aluminiumoxid besonders bevorzugt, insbesondere das für chromatographische Zwecke im Handel erhältliche "neutrale" Aluminiumoxid.

Die zweite Stufe des erfindungsgemässen Verfahrens wird vorteilhaft in Gegenwart von wenigstens zwei Äquivalenten einer starken Base durchgeführt. In diesem Fall entstehen die beiden Reaktionsprodukte Ethinylcyclopropan (I) und Methanol zunächst in der deprotonierten Form als Acetylid und Alkoholat. Das Acetylid wird bei der Aufarbeitung wieder zum Ethinylcyclopropan protoniert.

Besonders bevorzugt als starke Base sind Alkyllithiumverbindungen wie beispielsweise *n*-Butyllithium oder *tert*-Butyllithium.

Die folgenden Beispiele verdeutlichen die Durchführung des erfindungsgemässen Verfahren, ohne dass darin eine Einschränkung zu sehen ist.

### Beispiel 1

### (1,1-Dimethoxyethyl)cyclopropan (II)

Ein Gemisch aus 50 g (0,59 mol) Cyclopropylmethylketon, 82 g (0,77 mol) Trimethylorthoformiat, 200 ml Methanol und 0,25 g *p*-Toluolsulfonsäure wurde bei Raumtemperatur 2 h gerührt und anschliessend mit 0,15 g Natriummethanolat versetzt. Dann wurde unter vermindertem Druck zunächst das Methanol (bei ca. 400 mbar) und schliesslich (bei ca. 250 mbar) das Produkt (II) als farblose Flüssigkeit abdestilliert.

### Beispiel 2

### (1-Methoxyethenyl)cyclopropan (III)

Das (1,1-Dimethoxyethyl)cyclopropan (II) aus Beispiel 1 wurde mit 2 g Aluminiumoxid (neutral, 100-125 Mesh) versetzt und in einer Destillationsapparatur erwärmt, bis ein Gemisch aus Methanol und III bei 63 bis 94 °C überging. Dieses wurde in einer mit 40 ml Wasser und 40 ml Decalin beschickten Vorlage aufgefangen. Nach dem Ende der Reaktion wurden die beiden Phasen getrennt und die organische Phase mit Natriumsulfat getrocknet. Das Produkt (III) wurde durch Destillation isoliert.
Ausbeute: 30 g (51%, bezogen auf Cyclopropylmethylketon), farblose Flüssigkeit.
Kp.: 108-115 °C.

### Beispiel 3

### Ethinylcyclopropan (I)

140 ml (0,22 mol) einer 1.6 M Lösung von *n*-Butyllithium in Hexan wurden im Vakuum eingeengt. Dann wurde eine Lösung von 10 g (0,1 mol) (1-Methoxyethenyl)cyclopropan (III) in 60 ml Decalin zugegeben und das Gemisch 5 h auf 110 °C erhitzt. Anschliessend wurde es auf 0 °C abgekühlt und dann mit 50 ml Wasser versetzt. Die wässrige Phase wurde abgetrennt und die organische Phase über Natriumsulfat getrocknet. Das Produkt wurde durch Destillation isoliert.
Ausbeute: 2,6 g (39%), farblose Flüssigkeit.
Kp.: 50-51 °C.

## Patentansprüche

1. Verfahren zur Herstellung von Ethinylcyclopropan der Formel 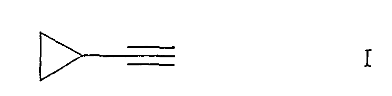 **dadurch gekennzeichnet, dass** (1,1-Dimethoxyethyl)cyclopropan der Formel 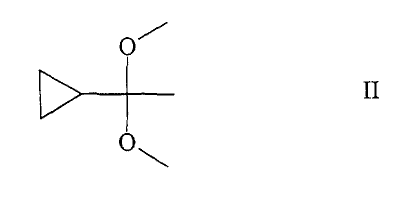 in einer ersten Stufe unter Abspaltung von Methanol zu (1-Methoxyethenyl)cyclopropan der Formel 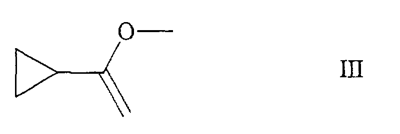 umgesetzt und in einer zweiten Stufe einer weiteren Methanolabspaltung unterzogen wird.

2. Verfahren nach Anspruch 1, **dadurch gekennzeichnet, dass** die erste Stufe unter heterogener Katalyse durchgeführt wird.

3. Verfahren nach Anspruch 2, **dadurch gekennzeichnet, dass** als heterogener Katalysator Aluminiumoxid eingesetzt wird.

4. Verfahren nach einem der Ansprüche 1 bis 3, **dadurch gekennzeichnet, dass** die zweite Stufe in Gegenwart von wenigstens zwei Äquivalenten einer starken Base durchgeführt wird.

5. Verfahren nach Anspruch 4, **dadurch gekennzeichnet, dass** als starke Base *n*-Butyllithium oder *tert*-Butyllithium eingesetzt wird.

## Claims

1. Process for the preparation of ethynylcyclopropane of the formula 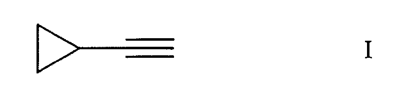 **characterized in that** (1,1-dimethoxyethyl)cyclopropane of the formula 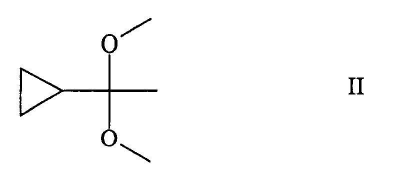 is reacted in a first step with elimination of methanol to give (1-methoxyethenyl)cyclopropane of the formula 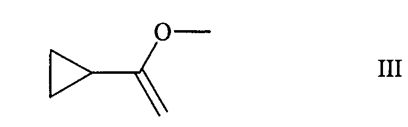 and in a second step is subjected to further methanol elimination.

2. Process according to Claim 1, **characterized in that** the first step is carried out under heterogeneous catalysis.

3. Process according to Claim 2, **characterized in that** the heterogeneous catalyst is aluminium oxide.

4. Process according to one of Claims 1 to 3, **characterized in that** the second step is carried out in the presence of at least two equivalents of a strong base.

5. Process according to Claim 4, **characterized in that** the strong base is *n*-butyllithium or *tert*-butyllithium.

## Revendications

1. Procédé de préparation d'éthynylcyclopropane de formule 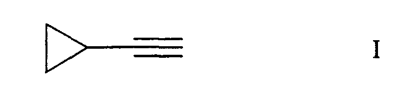 **caractérisé en ce que** le (1,1-diméthoxyéthyl)cyclopropane de formule 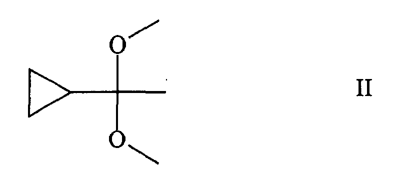 dans une première étape, est mis à réagir, en éliminant du méthanol, pour donner lieu au (1-méthoxyéthényl)cyclopropane de formule 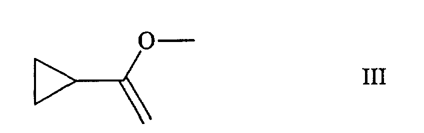 et est soumis, dans une deuxième étape, à une autre élimination de méthanol.

2. Procédé selon la revendication 1, **caractérisé en ce que** la première étape est réalisée sous catalyse hétérogène.

3. Procédé selon la revendication 2, **caractérisé en ce que** l'on utilise l'oxyde l'aluminium en tant que catalyseur hétérogène.

4. Procédé selon l'une quelconque des revendications 1 à 3, **caractérisé en ce que** la deuxième étape est réalisée en présence d'au moins deux équivalents d'une base forte.

5. Procédé selon la revendication 4, **caractérisé en ce que** l'on utilise le *n*-butyllithium ou le *tert*-butyllithium en tant que base forte.
